**Europäisches Patentamt**

**European Patent Office**

**Office européen des brevets**

(19)

(11) Veröffentlichungsnummer: **0 516 852 A1**

(12)
# EUROPÄISCHE PATENTANMELDUNG
## veröffentlicht nach Art. 158 Abs. 3 EPÜ

(21) Anmeldenummer: **91909047.2**

(22) Anmeldetag: **07.12.90**

(86) Internationale Anmeldenummer:
**PCT/SU90/00269**

(87) Internationale Veröffentlichungsnummer:
**WO 92/10451 (25.06.92 92/14)**

(51) Int. Cl.5: **C07C 11/08**, C07C 2/30

(43) Veröffentlichungstag der Anmeldung:
**09.12.92 Patentblatt 92/50**

(84) Benannte Vertragsstaaten:
**DE FR GB IT**

(71) Anmelder: **OKHTINSKOE NAUCHNO-PROIZVODSTVENNOE OBIENINENIE "PLASTOPOLIMER"**
**Poljustrovsky pr., 32**
**Leningrad 195197(SU)**

(72) Erfinder: **ZHUKOV, Viktor Ivanovich**
**ul. Neftezavodskaya, 4-1**
**Grozny, 364051(SU)**
Erfinder: **PROSKURIN, Vladimir Leonidovich**
**ul. Gudermesskaya, 96a-30**
**Grozny, 364015(SU)**
Erfinder: **KARTASHOVA, Natalia Vasilievna**
**mikroraion 8, 8-59**
**Budennovsk, 357920(SU)**
Erfinder: **IVOLGINA, Saida Rakhimovna**
**ul. Bogdana Khmelnitskogo, 23-2**
**Grozny, 364013(SU)**
Erfinder: **YATSENKO, Valery Alexeevich**
**pr. Lenina, 117-85**

**Grozny, 364051(SU)**
Erfinder: **KUDRINSKY, Alexandr Vasilievich**
**ul. Gurievskaya, 20-1 pos. Chernorechie**
**Grozny, 364046(SU)**
Erfinder: **IVANCHEV, Sergei Stepanovich**
**ul. Nalichnaya, 36-3-97**
**Leningrad, 199151(SU)**
Erfinder: **BELOV, Gennady Petrovich Shkolny bulvar, 5-53**
**Noginsky raion Moskovskaya obl.**
**pos. Chernogolovka, 142432(SU)**
Erfinder: **PETROV, Jury Maximovich**
**mikroraion 7, 17-37**
**Budennovsk, 357920(SU)**
Erfinder: **GERMASHEV, Anatoly Ivanovich**
**kvartal 175a, 5-5**
**mikroraion I**
**Budennovsk, 357920(SU)**

(74) Vertreter: **Hansen, Bernd, Dr. Dipl.-Chem. et al Hoffmann, Eitle & Partner Patent- und Rechtsanwälte Arabellastrasse 4 Postfach 81 04 20**
**W-8000 München 81(DE)**

(54) **VERFAHREN ZUR GEWINNUNG VON 1-BUTEN.**

(57) Das Verfahren zur Herstellung von Buten-(1) besteht darin, daß man die Dimerisation des Äthylens in einem kohlenwasserstoffhaltigen Lösungsmittel bei einer Temperatur von 50 bis 150°C und einem Druck von höchstens 4 mPa in Gegenwart eines katalytischen Systems durchführt. Den Bestand des katalytischen Systems bildet Tetraalkoxytitan, Trialkylaluminium und ein Modifikator. Man verwendet als Modifikator Dialkylaluminiumhalogen allgemeiner Formel $AlR_2Hal$, worin R für $C_2H_5$, iso-$C_4H_9$, Hal für Cl, Br, J steht, in Kombination mit einem aliphatischen oder zyklischen Äther, Das Molverhältnis Trialkylaluminium : Tetraalkoxytitan macht jeweils 2,5-7,0:1,0, das Molverhältnis Dialkylaluminiumhalogen : Tetraalkoxytitan jeweils 0,0l-1,7,:1,0, das Molverhältnis

aliphatischer oder zyklischer Äther : Tetraalkoxytitan jeweils 1,0-8,0:1,0 aus.

Die vorliegende Erfindung bezieht sich auf organische Chemie, insbesondere betrifft sie ein Verfahren zur Herstellung von Buten-(1).

Das Buten-(1) ist ein wertvoller Werkstoff zur Herstellung von Homopolymeren und verschiedenen Kopolymeren, bei deren Synthese an Buten-(1) strenge Anforderungen hinsichtlich des Gehalts von Mikrobeimengungen in demselben gestellt werden.

Das Buten-(1) kann durch Ausscheidung aus den $C_4$-Fraktionen von petrolchemischen Prozessen oder durch Dimerisation des Äthylens hergestellt werden.

Bei der Herstellung von Buten-(1) durch dessen Ausscheidung aus den genannten Fraktionen gewinnt man ein Zielprodukt mit einer großen Menge von Beimengungen, deswegen ist für seine Verwendung bei der Polymerisation und Kopolymerisation mit Olefinen ein drastisches System der Reinigung von den Azetylen- und Divinylverbindungen, von dem Kohlenstoffoxid- und -dioxid sowie von den Karbonyl- und Schwefelverbindungen des Sauerstoffs und Wassers erforderlich.

Bei der Herstellung von Buten-(1) durch Dimerisation des Äthylens ist der Gehalt an Beimengungen gering (insgesamt 10 $Mio^{-1}$).

Außerdem zeichnet sich dieser Prozeß durch eine hohe Stabilität aus.

Als Katalysatoren bei der Dimerisation von Äthylen können verschiedene Titan- und Zirkoniumverbindungen in Kombination mit aluminiumorganischen Verbindungen eingesetzt werden.

Die höchste buten-(1)-bezogene Selektivität besitzen Katalysatoren auf der Basis von Tetraalkoxytitanat, wobei allerdings folgende Probleme entstehen:

- der Verbrauch an Katalysator soll minimal sein;
- der Gehalt an Buten-(2), n-Butan und anfallenden Isohexenen soll minimal sein;
- die Ausbeute an sich bildenden hochmolekularen festen Nebenprodukten soll auch minimal sein, da deren Gehalt in einer Menge von 0,5 Masse% und mehr das Anhaften der Polymeren und das Verstopfen einzelner Ausrüstungsarten hervorruft.

Man führt beispielsweise nach einem der Verfahren durch Dimerisation des Äthylens in Gegenwart des Katalysators in einen Autoklaven diskontinuierlicher Wirkung zunächst einen vorher zubereiteten Katalysator ein. Man stellt den Katalysator durch Vermischen der Lösungen von $Ti(OC_4H_9)$ und $Al(C_2H_5)_3$ in Heptan bei einer Temperatur von 25°C innerhalb von 15 Minuten her. Dann führt man in den Autoklaven ein kohlenwasserstoffhaltiges Lösungsmittel - Heptan - mit einem Modifizierungsmittel, nämlich Isopropanol oder Butanol, bis zur Erzielung einer erforderlichen Konzentration ein. Danach wird dem Autoklaven Äthylen unter einem Druck von 0,4 mPa zugeführt und der Inhalt des Autoklaven wird auf eine Temperatur von 40 bis 50°C erhitzt. Die Dimerisation des Äthylens führt man innerhalb von 40 bis 90 Minuten durch, worauf die gewonnene, das Zielprodukt enthaltende Reaktionsmasse ausgeladen und die C -Fraktion (der Gehalt an Buten-(1) in derselben macht von 99,4 bis 99,7 Masse% aus) ausgeschieden wird.

Das Molverhältnis $Al(C_2H_5)_3$ : $Ti(OC_4H_9)_4$ macht 4,5:1 aus. Das Molverhältnis von Modifizierungsmitteln zu $Ti(OC_4H_9)_4$ beträgt 0,4:1 bis 0,7:1 (SU, A, 459451).

Bei der Durchführung dieses Verfahrens entstehen keine festen Nebenprodukte.

Der Gehalt an Buten-(2) und n-Butan in der $C_4$-Fraktion beträgt höchstens 0,5 bzw. höchstens 0,6 Masse%.

Somit macht das Verfahren es möglich, Buten-(1) mit einem ausreichenden Reinheitsgrad nach einer diskontinuierlichen Technologie herzustellen.

Bei der Durchführung des Verfahrens nach einer kontinuierlichen Variante ist es unmöglich, Qualitätskennwerte des Prozesses zu erzielen. Während der Dimerisation kommt es zur Bildung eines festen Polymeren, dessen Gehalt in der Reaktionsmasse höchstens 2,3 Masse%, beträgt; in der $C_4$-Fraktion wird der Gehalt an Buten-(2) und n-Butan auf 0,8 bzw. 1,5 Masse% gesteigert.

Bekannt ist noch ein Verfahren zur Herstellung von Buten-(1) durch Dimerisation des Äthylens (SU, A, 455082). Dieses Verfahren realisiert man auch diskontinuierlich nach der oben beschriebenen Methodik unter Ausnutzung des in dieser angegebenen Katalysators. Als Modifikator verwendet man Diisopropyläther oder Dioxan und führt man die Dimerisation unter einem Druck von 0,2 bis 1,5 mPa bei einer Temperatur von 70 bis 80°C durch. Der Gehalt der $C_4$-Fraktion an Buten-(1) beträgt 99,2-99,4 Masse%. Bei der Durchführung dieses Verfahrens entstehen ebenfalls keine feste Nebenprodukte. Der Gehalt der $C_4$-Fraktion an Buten-(2) und n-Buten beträgt höchstens 0,45 bzw. 0,53 Masse%.

Die Durchführung dieses Prozesses nach der kontinuierlichen Variante ist unmöglich infolge der Bildung eines festen Polymeren, dessen Gehalt in der Reaktionsmasse höchstens 2,5 Masse% ausmacht.

Dabei wird der Gehalt der $C_4$-Fraktion an Buten-(2) und n-Buten bis auf 1,2 bzw. 1,5 Masse% gesteigert.

Aus diesem Grunde ist es bei der Realisierung der oben genannten Verfahren in der kontinuierlichen Variante notwendig, in das technologische Schema eine komplizierte Baueinheit zur Isolierung des Polymeren einzuschließen sowie eine besondere Baueinheit zum Herausführen des Buten-(2) und des n-Butans aus der $C_4$-Fraktion zu fertigen. Dies verteuert starr den Prozeß und macht diesen nicht konkurrenzfähig.

Der vorliegenden Erfindung liegt die Aufgabe zugrunde, solch ein Verfahren zur Herstellung von Buten-(1) zu entwickeln, das es ermöglicht, ein Produkt mit einem hohen Reinheitsgrad in einem kontinuierlichen Verfahren nach einer einfachen Technologie zu erhalten.

Diese Aufgabe wird dadurch gelöst, daß ein solches Verfahren zur Herstellung von Buten-(1) durch Dimerisation des Äthylens in einem kohlenwasserstoffhaltigen Lösungsmittel in Gegenwart eines katalytischen Systems, das aus Tetraalkoxytitan, Trialkylaluminium und aus einem Modifikator besteht, bei einer Temperatur von 50 bis 150°C und einem Druck von höchstens 4 mPa vorgeschlagen wird, bei dem erfindungsgemäß als Modifikator Dialkylaluminiumhalogen der allgemeinen Formel $AlR_2Hal$, worin R, für $C_2H_5$, iso-$C_4H_9$, Hal für Cl, Br, J steht, in Kombination mit einem aliphatischen oder zyklischen Äther verwendet wird, wobei das Molverhältnis Trialkylaluminium:Tetraalkoxytitan jeweils 2,5:7,1, das Molverhältnis Dialkylaluminiumhalogen:Tetraalkoxytitan jeweils 0,01-1,7:1 und das Molverhältnis aliphatischer oder zyklischer Äther: :Tetraalkoxytitan jeweils 1,0-8,0:1 ausmacht.

Man kann als kohlenwasserstoffhaltiges Lösungsmittel beispielsweise Hexan- und Heptanfraktionen in einem schmalen Temperaturbereich des Aussiedens, verschiedene aromatische Kohlenwasserstoffe (Benzol, Toluol), Buten-(1), Isohexene oder Gemische der Genannten Kohlenwasserstoffe, genommen in verschiedenen Verhältnissen, verwenden.

Wie oben ausgeführt wurde, erfolgt die Dimerisation des Äthylens bei einer Temperatur von 50 bis 150°C. Der gewählte Temperaturbereich ist optimal, da bei einer unterhalb 50°C liegenden Temperatur der Vorgang bei niedrigen Geschwindigkeiten verläuft und bei einer oberhalb 150°C liegenden Temperatur die Ausbeute an Nebenprodukten schroff zunimmt und die Aktivität des Katalysators vermindert wird, da sich unter diesen Bedingungen der Katalysator, insbesondere eine Titanverbindung, teilweise zersetzt.

Der gewählte Druckbereich von höchstens 4 MPa bewirkt eine ausreichende Geschwindigkeit der Reaktion, Eine Drucksteigerung über 4 mPa führt zu keiner weiteren Zunahme der Reaktionsgeschwindigkeit.

Zum katalytischen System gehört Tetraalkoxytitan, Trialkylaluminium und Modifikator. Tetraalkoxytitan und Trialkylaluminium sind konventionnelle Komponenten bei der Synthese von Buten-(1) durch Dimerisation des Äthylens. Als Modifikator verwendet man Dialkylaluminiumhalogen allgemeiner Formel Al $R_2Hal$, worin R für $C_2H_5$, iso-$C_4H_9$, Hal für Cl, Br, J steht, in Kombination mit einem aliphatischen oder Zyklischen Äther. Die Verwendung solch eines Systems der Modifikatoren im Bestand des Katalysators ermöglicht es, den Reduktionsgrad von Titanverbindungen zu regeln, ohne dabei die Bildung der Niedrigvalenzform desselben ($Ti^{+3}$, $Ti^{+2}$) zuzulassen, die zur Bildung von Hebenprodukten und zur Isomerisation des Buten-(1) führen.

Die Molverhältnisse der Komponenten sind von großer Bedeutung.

Man nimmt das Molverhältnis von Trialkylaluminium zu Tetraalkoxytitan in einem Bereich von 2,5-7:1. Eine Senkung desselben unter 2,5:1 bringe eine schroffen Herabsetzung der Reaktionsgeschwindigkeit mit sich, während eine Steigerung des Molverhältnisses über 7:1 zur Vergrößerung des Verbrauchs an Komponenten des Katalysators und der Ausbeute an Webenprodukten führt.

Das gewählte Molverhältnis Dialkylaluminiumhalogen:Tetraalkoxytitan reguliert die Ausbeute am Polymeren und Buten-(2). Bei einer Größe des Verhältnisses unter 0,01:1 übt Dialkylaluminiumhalogen keinen Einfluß auf die Ausbeute am Polymeren und an Buten-(2) aus. Bei einem größeren Verhältnis als 1,7:1 kommt es zur Herabsetzung der Prozeßgeschwindigkeit.

Aliphatische und zyklische Äther bilden Komplexe mit Tetraalkoxytitan und Trialkylaluminium, indem sie dabei den Reduktionsgrad des Tetraalkoxytitans regulieren und die Reduktionsfähigkeit des Trialkylaluminiums herabsetzen, was die Stabilität des katalytischen Systems während der Dimerisation bewirkt.

Bei einem Molverhältnis gewählter Äther:Tetraalkoxytitan weniger als 1,0:1,0 zeigt sich kein Effekt der Stabilisation des Systems. Bei einem Molverhältnis mehr als 8,0:1 wird ein starker Abfall der Dimerisationsgeschwindigkeit beobachtet.

In einzelnen Fällen führt man die Dimerisation des Äthylens in Gegenwart von Wasserstoff in einer Menge von 1 bis 200 Mol je 1 Mol Tetraalkoxytitan durch.

Bei der Dimerisation ruft der Wasserstoff den Abbruch der Polymerenketten der sich bildenden hochmolekularen Nebenprodukte hervor, wobei deren Molekularmasse vermindert und dadurch die Menge des entstehenden Polymeren herabgesetzt wird.

Bei einer Wasserstoffmenge unter I Mol je I Mol Tetraalkoxytitan wird der genannte Effekt nicht nachgewiesen, während eine Vergrösserung dar Wasserstoffmenge auf mehr als 200 Mol zum Hydrieren des Buten-(I) führt.

Als aliphatischen Äther empfiehlt es sich, beispielsweise Diäthyl-, Dipropyl- Dibutyl- oder Diisoamyläther und als zyklischen Äther beispielsweise Dioxan oder Tetrahydrofuran zu verwenden.

Das erfindungsgemäße Verfahren zur Herstellung von Buten-(1) durch Dimerisation des Äthylens ermöglicht es, bei kontinuierlichem Verfahren das Buten-(1) mit einem Gehalt an Mikrobeimengungen von 10 Mio$^{-1}$ bei einer einfachen apparativen Gestaltung herzustellen.

Als Katalysator- und Modifikatorkomponenten kommen handelsübliche Produkte in Farge,

Das erfindungsgemäße Verfahren ist wirtschaftlich, ökologisch rein, die erhaltenen Nebenprodukte - Isoxene - lassen sich leicht verwerten, die Katalysatorrückstände, beispielsweise Modifikatoren, deren Menge insgesamt 0,5-1,5 kg/1 t $C_4H_8$ nicht überschreitet, werden verbrannt. Die Abwässer fehlen.

Das Verfahren zur Herstellung von Buten-(1) durch Dimerisation von Äthylen ist in technologischer Ausführung einfach und wird wie folgt durchgeführt.

Zur Herstellung des Katalysators werden seine Ausgangskomponenten, Tetraalkoxytitan und Trialkylaluminium, in, verschiede Behälter eingebracht. Danach führt man jedem der Behälter Modifikatoren zu. Die Konzentration jeder der Komponenten wird mit Hilfe eines Lösungsmittels auf die erforderliche Konzentration gebracht. Die zubereiteten Komponenten des katalytischen Systems werden mittels der Dosierpumpen unmittelbar dem Reaktor oder zum vorläufigen Vermischen und dann dem Reaktor zugeführt. In den Reaktor wird unter erforderlichem Druck Äthylen und notwendigerfalls Wasserstoff eingeführt. Die Temperatur des Prozesses wird mit verschiedenen bestehenden Verfahren zur Abnahme der Reaktionswärme aufrechter halten . Die durch Dimerisation des Äthylens gewonnene Reaktionsmasse, die eine geringe Menge von Äthylen, Buten-(1), Isohexenen, Lösungsmittel und verbrauchtem Katalysator enthält, wird zur Trennung geleitet. Die Trennung der Produkte wird in an sich bekannter Weise, beispielsweise durch Rektifikation, durchgeführt.

Zum besseren Verständnis der vorliegenden Erfindung werden folgende konkrete Beispiele angeführt.

Beispiel 1

In einem Reaktor von 1 m³ Fassungsvermögen führt man kontinuierlich einen Katalysator ein. Der Katalysator stellt ein katalytisches System folgender Zusammensetzung mit einer Zuführgeschwindigkeit dar:

| | |
|---|---|
| 0,2 kg/h | Tetrabutoxytitan |
| 0,28 kg/h | Triäthylaluminium |
| 0,027 kg/h | Diäthylaluminiumchlorid |
| 0,093 kg/h | Diisoamyläther. |

Dem Reaktor führt man auch kontinuierlich mit einer Geschwindigkeit von 35 kg/h Hexanfraktion mit Sidegrenzen von 64 bis 68°C zu. Das Molverhältnis der Komponenten ist wie folgt:

| | |
|---|---|
| Triäthylaluminium : Tetrabutoxytitan | 0,4:1 |
| Diäthylaluminiumchlorid: Tetrabutoxytitan | 0,4:1 |
| Diisoamyläther : Tetrabutoxytitan | 1:1. |

Man führt dem Reaktor auch 1,16 kg/h $H_2$ zu. Das Molverhältnis $H_2$ : $Ti(OC_4H_9)$ = 1:1:

Man führt den Prozeß bei einer Temperatur von 80°C durch. In den Reaktor führt man kontinuierlich Äthylen ein und unterhält den Druck von 1;0 mPa; die Menge des umgesetzten Äthylens macht 100 kg/h aus. Die mittlere Verweilzeit der Reaktionsmasse in der Reaktionszone beträgt 4 Stunden.

Die erhaltene Reaktionsmasse wird zur Trennung kontinuierlich aus dem Reaktor ausgetragen. Man erhält eine $C_4$-Fraktion in einer Menge von 93 Masse%. Der Gehalt der Fraktion an Buten-(1) macht 99,4 Masse%, an Buten-(2) 0,1 Masse% und an n-Butan 0,4 Masse% aus. Die Ausbeute an Polymerem beträgt 0,4 Masse%, darunter Metallverbindungen, umgerechnet auf Oxide.

Beispiel 2

Man stellt Buten-(1) nach der in Beispiel 1 beschriebenen Methodik her, mit Ausnahme dessen, daß man die Dimerisation in Abwesenheit von Wasserstoff durchführt.

Man erhält eine $C_4$-Fraktion in einer Menge von 93,1 Masse%. Der Gehalt derselben an Buten-(1) macht 99,5 Masse%, der an Buten-(2) 0,1 Masse% und der an n-Butan 0,3 Masse% aus. Die Ausbeute an Polymerem beträgt 0,45 Masse%.

Beispiel 3

In einen Reaktor von 1 m³ Fassungsvermögen führt man kontinuierlich einen Katalysator folgender Zusammensetzung mit einer Geschwindigkeit ein:

| 0,2 kg/h | Tetrabutoxytitan |
|----------|------------------|
| 0,235 kg/h | Triäthylaluminium |
| 0,121 kg/h | Diäthylaluminiumchlorid |
| 0,150 kg/h | Diisoamyläther. |

Dem Reaktor führt man mit einer Geschwindigkeit von 40 kg/h Heptanfraktion mit Siedergrenzen von 92 bis 98°C zu.
Das Molverhältnis der Komponenten des Katalysators ist wie folgt:

| Triäthylaluminium : Tetrabutoxytitan | 3,5:1,0 |
|--------------------------------------|---------|
| Diäthylaluminiumchlorid : Tetrabutoxytitan | 1,7:1,0 |
| Diisoamyläther : tetrabutoxytitan | 1,55:1,0. |

Man führt in den Reaktor kontinuierlich Wasserstoff in einer Menge von 11,7 kg/h ein. Das Molverhältnis $H_2$ : Tetrabutoxytitan beträgt 10:1 .
Man führt den Prozeß bei einer Temperatu von 150°C durch. In den Reaktor führt man kontinuierlich Äthylen mit einer Geschwindigkeit von 120 kg/h ein und unterhält den Druck von 1,0 mPa. Die mittlere Verweilzeit der Reaktionsmasse in der Reaktionszone beträgt 4 Stunden. Die Reaktionsmasse wird kontinuierlich zur Trennung ausgetragen.
Die Ausbeute an $C_4$-Fraktion beträgt 74,5 Masse%. Die Zusammensetzung der Fraktion : Buten-(1) - 99,4 Masse%, Buten-(2) - 0,4 Masse%, n-Butan - 0,1 Masse%. Die Ausbeute an Polymerem macht 0,08 Masse% aus.

Beispiel 4

In einen Reaktor von 1 m³ Fassungsvermögen führt man kontinuierlich einen Katalysator folgender Zusammensetzung mit einer Geschwindigkeit ein:

| 0,2 kg/h | Tetrabutoxytitan |
|----------|------------------|
| 0,168 kg/h | Triäthylaluminium |
| 0,0007 kg/h | Diäthylaluminiumchlorid |
| 0,178 kg/h | Tetrahydrofuran. |

Dem Reaktor führt man kontinuierlich Buten-Hexen-Gemisch als Lösungsmittel (80 Masse% Buten-(1), das Übrige - Isohexene) zu. Die Zuführgeschwindigkeit macht 40 kg/h aus.
Das Molverhältnis der Komponenten des Katalysators ist wie folgt:

| Triäthylaluminium : Tetrabutoxytitan | 2,5:1,0 |
|--------------------------------------|---------|
| Diäthylaluminiumchlorid : Tetrabutoxytitan | 0,01:1,0 |
| Tetrahydrofuran : Tetrabutoxytitan | 3,5;1,0. |

Man führt den Prozeß bei einer Temperatur von 50°C durch.
Man führt Äthylen mit einer Geschwindigkeit von 105 kg/h kontinuierlich ein. Der Druck beträgt 1,0 mPa. Die mittlere Verweilzeit macht 4 Stunden aus. Die Reaktionsmasse wird aus dem Reaktor kontinuierlich ausgetragen. Die Ausbeute an $C_4$-Fraktion ist 93,5 Masse% gleich. Die Zusammensetzung der Fraktion: Buten-(1) - 99,1 Masse%, Buten-(2) - 0,05 Masse%, n-Butan - 0,25 Masse%, Polymeres - 0,004 Masse%.

6

EP 0 516 852 A1

Beispiel 5

In einen Reaktor von 1 m³ Fassungsvermögen führt man kontinuierlich einen Katalysator folgender Zusammensetzung mit einer Geschwindigkeit ein:

| | |
|---|---|
| 0,061 kg/h | Tetrabutoxytitan |
| 0,156 kg/h | Triäthylaluminium |
| 0,003 kg/h | Diäthylaluminiumchlorid |
| 0,100 kg/h | Tetrahydrofuran. |

Dem Reaktor 10 kg/h führt man Toluol als Lösungsmittel kontinuierlich zu. Das Molverhältnis der Komponenten des Katalysators ist wie folgt:

| | |
|---|---|
| Triäthylaluminium : Tetrabutoxytitan | 7,0:1,0 |
| Diäthylaluminiumchlorid : Tetrabutoxytitan | 0,1:1,0 |
| Tetrahydrofüran : Tetrabutoxytitan | 8,0:1,0. |

Man führt in den Reaktor Äthylen in einer Menge von 101 kg/h kontinuierlich ein. Man führt den Prozeß bei einer Temperatur von 60°C durch. Der Druck beträgt 2,0 mPa. Die mittlere Verweilzeit der Reagenzien in dem Reaktor macht 12 Stunden aus.

Die Reaktionsmasse wird aus dem Reaktor kontinuierlich ausgetragen. Die Ausbeute an $C_4$-Fraktion beträgt 94 Masse%. Die Zusammensetzung der Fraktion: Butan-(1) - 99,8 Masse%, Buten-(2) - unter 0,02 Masse%, n-Butan - 0,18 Masse%. Die Ausbeute an Polymerem macht 0,003 Masse% aus.

Beispiel 6

In einen Reaktor von 1 m³ Fassungsvermögen führt man kontinuierlich einen Katalysator folgender Zusammensetzung mit einer Zuführgeschwindigkeit ein:

| | |
|---|---|
| 0,2 kg/h | Tetrapropoxytitan |
| 0,63 kg/h | Triisobutylaluminium |
| 0,043 kg/h | Diisobutylaluminiumbromid |
| 0,21 kg/h | Diäthyläther. |

Dem Reaktor führt man auch 30 kg/h Benzol kontinuierlich zu.
Dag Molverhältnis der Komponenten ist wie folgt:

| | |
|---|---|
| Triisobutylaluminium : Tetrapropoxytitan | 4,5:1,0 |
| Diisobutylaluminiumbromid : Tetrapropoxytitan | 0,5:1,0 |
| Diäthyläther : Tetrapropoxytitan | 4,0:1,0. |

Man führt in den Reaktor Äthylen kontinuierlich ein und unterhält den Druck von 1,0 mPa. Der Prozeß wird bei einer Temperatur von 70°C durchgeführt.

Die Menge des umgesetzten Äthylens beträgt 110 kg/h. Die mittlere Verweilzeit der Reaktionsmasse in der Reaktionszone macht 1 Stunde aus.

Man erhält eine $C_4$-Fraktion in einer Menge von 93,5 Masse%. Der Gehalt derselben an Buten-(1) beträgt 99,5 Masse%, der an Buten-(2) - 0,2 Masse% und der an n-Butan - 0,2 Masse%. Die Ausbeute an Polymerem macht 0,35 Masse% aus.

Beispiel 7

In einen Reaktor von 1 m³ Fassungsvermögen führt man kontinuierlich einen Katalysator folgender Zusammensetzung mit einer Zuführgeschwindigkeit ein:

7

| | |
|---|---|
| 0,2 kg/h | Tetrapentoxytitan |
| 0,36 kg/h | Diisobutylaluminiumhydrid |
| 0,22 kg/h | Diäthylaluminiumjodid |
| 0,14 kg/h | Dioxan. |

Dem Reaktor führt man auch als Lösungsmittel eine Buten-Hexan-Fraktion (30 Masse% - Buten-(1), alles übrige - Isohexene) mit einer Zuführgeschwindigkeit von 35 kg/h kontinuierlich zu.

Das Molverhältnis der Komponenten ist wie folgt:

| | |
|---|---|
| Diisobutylalumoniumhydrid : Tetrapentoxytitan | 5,0:1,0 |
| Diäthylaluminiumjodid : Tetrapentoxytitan | 0,2:1,0 |
| Dioxan : Tetrapentoxytitan | 3,0:1,0. |

Man führt den Prozeß bei einer Temperatur von 80°C und bei einem Druck von 2,0 mPa durch. Die Menge des umgesetzten Äthylens macht 160 kg/h aus. Die mittlere Verweilzeit der Reaktionsmasse in der Reaktionszone beträgt 2 Stunden.

Man ehrält eine $C_4$-Fraktion in einer Menge von 92,5 Masse%. Der Gehalt derselben an Buten-(1) beträgt 99,2 Masse%, der an Buten-(2) - 0,30 Masse% und der an n-Butan - 0,35 Masse%. Die Ausbeute an Polymerem ist 0,4 Masse% gleich.

Beispiel 8

In einen Reaktor von 1 m³ Fassungsvermögen führt man kontinuierlich einen Katalysator folgender Zusammensetzung mit einer Zuführgeschwindigkeit ein:

| | |
|---|---|
| 0,2 kg/h | Tetrabutoxytitan |
| 0,3 kg/h | Triäthylaluminium |
| 0,007 kg/h | Diäthylaluminiumchlorid |
| 0,06 kg/h | Dipropyläther |
| 0,236 kg/h | Wasserstoff . |

Dem Reaktor führt man auch Benzol mit einer Zuführgeschwindigkeit von 25 kg/h kontinuierlich zu.

Das Molverhältnis der Komponenten ist wie folgt:

| | |
|---|---|
| Triäthylaluminium : Tetrabutoxytitan | 4,5:1,0 |
| Diäthylaluminiumchlorid : Tetrabutoxytitan | 0,1:1,0 |
| Dipropyläther : Tetrabutoxytitan | 1,0:1,0 |
| Wasserstoff : Tetrabutoxytitan | 200,0:1,0. |

Man führt den Prozeß bei einer Temperatur von 80°C durch.

Man führt in den Reaktor Äthylen kontinuierlich ein und unterhält den Druck von 1,0 mPa, die Menge des umgesetzten Äthylens beträgt 125 kg/h. Die mittlere Verweilzeit der Reaktionsmasse in der Reaktionszone macht 2 Stunden aus.

Man erhält eine $C_4$-Fraktion in einer Menge von 99,6 Masse%. Der Gehalt derselben an Bunten-(1) macht 99,8 Masse%, der an Buten-(2) - 0,1 Masse% und der an n-Butan 0,1 Masse% aus. Das Polymere fehlt.

Beispiel 9

In einen Reaktor von 1 m³ Fassungsvermögen fuhrt man kontinuierlich einen Katalysator folgender Zusammensetzung mit einer Geschwindigkeit ein:

| | |
|---|---|
| 0,2 kg/h | Tetrabutoxytitan |
| 0,2 kg/h | Triäthylaluminium |
| 0,18 kg/h | Diäthylaluminiumchlorid |
| 0,3 kg/h | Dibutyläther . |

Dem Reaktor führt man auch 30 kg/h Heptan zu.
Das Molverhältnis der Komponenten ist wie folgt:

| | |
|---|---|
| Triäthylaluminium : Tetrabutoxytitan | 3,0:1,0 |
| Diäthylaluminiumchlorid : Tetrabutoxytitan | 2,5:1,0 |
| Dibutyläther : Tetrabutoxytitan | 4,0:1,0. |

Man führt in den Reaktor Äthylen kontinuierlich ein und unterhält den Druck von 4,0 mPa. Der Prozeß wird bei einer Temperatur von 60°C durchgeführt. Die Menge des umgesetzten Äthylens beträgt 310 kg/h. Die mittlere Verweilzeit der Reaktionsmasse in der Reaktionszone macht 1 Stunde aus.

Man erhält eine $C_4$-Fraktion in einer Menge von 99,8 Masse%. Der Gehalt derselben an Buten-(1) macht 99,8 Masse% und der an n-Butan -0,2 Masse% aus, Buten-(2) - - fehlt. Die Ausbeute an Polymerem beträgt 0,09 Masse%.

**Patentansprüche**

1. Verfahren zur Herstellung von Buten-(1) durch Dimerisation des Äthylens in einem kohlenwasserstoffhaltigen Lösungsmittel in Gegenwart eines katalytischen Systems, das aus Tetraalkoxytitan, Trialkylaluminium und aus einem Modifikator besteht, bei einer Temperatur von 50 bis 150°C und einem Druck von höchstens 4 mPa, dadurch **gekennzeichnet,** daß man als Modifikator-Dialkylaluminiumhalogen der allgemeinen Formel $AlR_2Hal$, worin R für $C_2H_5$,iso-$C_4H_9$, Hal für Cl, Br, J steht, in Kombination mit einem aliphatischen oder zyklischen Äther verwendet, wobei das Molverhältnis Trialkylaluminium : Tetraalkoxytitan jeweils 2,5-7,0:1,0, das Molverhältnis Dialkylaluminiumhalogen : Tetraalkoxytitan jeweils 0,01-1,7:1,0 und das Molverhältnis aliphatischer oder zyklischer Äther : Tetraalkoxytitan jeweils 1,0-8,0:1,0 ausmacht.

2. Verfahren nach Anspruch 1, dadurch **gekennzeichnet,** daß man die Dimerisation des Äthylens auch in Gegenwart von Wasserstoff in einer Menge von 1 bis 200 Mol je 1 Mol Tetraalkoxytitan durchführt.

3. Verfahren nach Anspruch 1, dadurch **gekennzeichnet**, daß man als aliphatischen Äther Diäthyl-, Dipropyl-, Dibutyl- oder Diisoamyläther verwendet.

4. Verfahren nach Anspruch 1, dadurch **gekennzeichnet**, daß man als zyklischen Äther Dioxan oder Tetrahydrofuran verwendet.

# INTERNATIONAL SEARCH REPORT

International Application No PCTT/SU 90/00269

**I. CLASSIFICATION OF SUBJECT MATTER (if several classification symbols apply, indicate all) [6]**

According to International Patent Classification (IPC) or to both National Classification and IPC

IPC[5] C 07 C 11/08, 2/30

**II. FIELDS SEARCHED**

| Minimum Documentation Searched [7] | |
|---|---|
| Classification System | Classification Symbols |
| IPC[5] | C 07 C 2/30, 11/08 |

Documentation Searched other than Minimum Documentation
to the Extent that such Documents are Included in the Fields Searched [8]

**III. DOCUMENTS CONSIDERED TO BE RELEVANT [9]**

| Category [*] | Citation of Document, [11] with indication, where appropriate, of the relevant passages [12] | Relevant to Claim No. [13] |
|---|---|---|
| A | FR, A1, 2552080 (INSTITUT FRANCAIS DU PETROLE), 22 March 1985 (22,03.85), example 6, the claims | 1,3 |
| A | FR, A1, 2552079 (INSTITUT FRANCAIS DU PETROLE) 22 March 1985 (22.03.85) page 3, lines 4-6,16-22, claims 1,2,6 | 1,4 |
| A | DE, A1, 3301162 (EC ERDÖLCHEMIE GMBH), 19 July 1984 (19.07.84), the claims | 1 |
| A | SU, A1, 459451 (Zhukov V.I. et al) 21 April 1975 the claims | 1 |
| A | SU, A1, 455082 (Zhukov V.I. et al) 15 April 1975 (15.04.75) the claims, (cited in the description) | 1 |
| A | SU, A1, 493454 (Filial Instituta Khimisheskoi fiziki AN SSSR) 2 July 1976 (02.07.76), the claims | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 23 July 1991 (23.07.91) | 28 August 1991 (28.08.91) |
| International Searching Authority | Signature of Authorized Officer |
| ISA/SU | — |